# EUROPEAN PATENT SPECIFICATION

(11) **EP 3 206 578 B1**
(45) Date of publication and mention of the grant of the patent: **21.12.2022**
(21) Application number: 14799055.0
(22) Date of filing: 16.10.2014
(51) Int. Cl.: A61M 16/10, A61B 5/08, A61M 16/00, A61M 16/12

(54) **MODULAR MONITORING AND VENTILATION SYSTEM**
MODULARES ÜBERWACHUNGS- UND BELÜFTUNGSSYSTEM
SYSTÈME MODULAIRE DE SURVEILLANCE ET DE VENTILATION

(43) Date of publication of application: 23.08.2017
(73) Proprietor: Maquet Critical Care AB, 171 06 Solna (SE)
(72) Inventor: AHLMÉN, Christer, S-192 51 Sollentuna (SE); LONCAR, Mario, S-178 39 Ekerö (SE); EMTELL, Pär, S-162 45 Vällingby (SE); KOCK, Mikael, S-184 91 Åkersberga (SE)
(74) Representative: Zacco Sweden AB
(86) International application number: PCT/SE2014/051223
(87) International publication number: WO 2016/060596

(56) References cited:
- WO-A1-2009/082295
- WO-A1-2013/027151
- US-A1- 2006 213 518
- US-A1- 2009 326 389
- US-A1- 2012 298 108

## Description

### TECHNICAL FIELD

The present invention relates to a standalone monitor unit for monitoring patient-related parameters indicative of the physiological status of a patient, which monitor unit is configured to receive at least one signal indicative of such a patient-related parameter from at least one sensor, and to display information related to said signal on a display of the monitor unit.

### BACKGROUND

Medical monitors for monitoring patient-related parameters of patients undergoing intensive care are well known in the art.

This disclosure relates to the standalone-type of medical monitors, i.e. to self-contained medical monitors not being integrated in other medical devices, such as ventilators, anaesthesia machines or the like.

Such standalone monitors are often used in intensive care units and operating rooms to collect, analyse and display information related to patient-related parameters measured by various sensors to which the monitor unit is connected via a plurality of signal inputs of the monitor unit. Non-exclusive examples of patient-related parameters sometimes monitored by means of such standalone monitor units are ECG, heart rate, blood pressure and blood oxygen saturation.

The portable monitor disclosed in EP0735498 is an example of a standalone monitor for acquiring medical data from a plurality of sensors adapted for attachment to a patient.

EP 1702649 discloses another type of display-equipped base station constituting a monitor unit for monitoring patient-related parameter. The base station is configured for data exchange with a therapy device, e.g. in form of a ventilator device, to which the monitor unit is connectable.

US 2021/298108 A1 discloses a system comprising one or more patient monitors to detect physiological conditions of a patient. The physiological conditions can be used to monitor the patient and/or control the operation of a ventilator. The monitor may be a diaphragm electromyographic (EMG) detection system that detects the EMG signals produced by the diaphragm during breathing.

Standalone monitors are often configured to automatically generate an alarm if a monitored patient-related parameter deviates too much from a predefined normal value or reference value.

When such an alarm is generated the patient is often critically ill and in urgent need of sophisticated ventilatory treatment. Typically, this means that the patient has to be moved from the intensive care unit in which the patient has been monitored to a ventilator capable of providing the required ventilatory treatment, or that a ventilator has to be moved to the intensive care unit in which the patient is monitored and connected to the patient for the provision of the ventilatory treatment. Both scenarios imply bulky transportation as well as time-consuming disconnection and reconnection of various types of sensors and/or patient connectors. Since the time to start of ventilatory treatment is crucial to the outcome of the treatment, there is a desire to solve or at least mitigate at least one of the above mentioned problems of monitoring systems according to prior art.

### SUMMARY OF THE INVENTION

It is an object of the invention to provide a solution that solves or at least mitigates one or more of the above mentioned problems.

It is another object of the invention to provide a solution that minimizes the time to provision of adequate ventilatory treatment of a patient connected to a standalone monitor unit, after said monitor unit has indicated the need for such ventilatory treatment. It is another object of the invention to provide a monitoring and ventilation system that is particularly suitable for use in monitoring and ventilation of neonatal patients.

These and other objects are achieved by a modular monitoring and ventilation system according to claim 1.

The modular monitoring and ventilation system of the present invention comprises two separate physical and functional units configured to provide some basic functionality when operated separately, i.e. as separate units, and some additional functionality when communicatively connected to each other to allow exchange of information between the two units.

The first unit is a standalone monitor unit for monitoring patient-related parameters indicative of the physiological status of a patient. To this end, the standalone monitor unit comprises at least one signal input through which it receives at least one signal indicative of such a patient-related parameter from at least one sensor. The monitor unit is further configured to display information related to said signal on a display of the monitor unit, e.g. in form of a signal curve constituting a graphical representation of said signal, or a numeric value of the patient-related parameter of which said signal is indicative.

The second unit is a standalone pneumatic unit which, when operated alone as a separate unit, is capable of providing only basic ventilatory treatment to the patient. In one embodiment, the pneumatic unit is a small-sized standalone CPAP device which, when operated as a separate unit, is capable of providing nothing but CPAP (continuous positive airway pressure) therapy and, additionally, high flow oxygen therapy to a patient. In this context, small-sized means that pneumatic unit is a portable unit which is considerably smaller in size than a conventional full scale ventilator.

The standalone monitor unit and the standalone pneumatic unit are configured to be put in a paired state in which they are communicatively connected to each other in order to exchange information, e.g. monitored patient parameters, setting parameters, operational parameters or any other information related to the patient or the operation of any or both of the two units. In this paired state, the monitor unit and the pneumatic unit are further configured to cooperate to provide ventilatory treatment to the patient by controlling the operation of the pneumatic unit based on the at least one signal received by the monitor unit from the at least one sensor and indicative of said monitored patient-related parameter.

This means that the operation of the pneumatic unit, in the paired state, is controlled based on said at least one patient-related parameter monitored by the monitor unit, so as to adjust the ventilatory treatment provided by the pneumatic unit to the current needs of the patient, as indicated by said patient-related parameter.

To this end, the monitor unit is configured to transmit said signal received from the at least one sensor or a signal derived from said received signal to the pneumatic unit, whereby the pneumatic unit is configured to receive and use the transmitted signal as a control signal in the control of the operation of the pneumatic unit.

Controlling the operation of the pneumatic unit based on a parameter or a control signal herein means that at least the supply of breathing gas supplied to the patient by the pneumatic unit is controlled based on said parameter or control signal, typically by regulating the flow of breathing gas supplied to the patient in order for a measured flow and/or pressure to follow a desired flow and/or pressure curve calculated based on said parameter or control signal.

The standalone monitor unit of the present disclosure thus constitutes a new type of therapy-enabling monitor unit which can be quickly and easily adapted to provide ventilatory treatment to the monitored patient by communicatively connecting the monitor unit to a pneumatic unit so as to form a modular monitoring and ventilation system allowing ventilation of the patient to be made based on parameters monitored by the monitor unit.

Since efficient ventilation of the patient based on monitored patient-related parameters can be achieved by pairing the monitor unit with the pneumatic unit, the need for moving the patient from the monitoring site to a ventilator in case the need for ventilatory treatment arises is eliminated. Instead, in case the need for ventilatory treatment arises, a small-sized, standalone pneumatic unit is paired with the standalone monitor unit to enable ventilatory treatment based on monitored patient-related parameters to be carried out on site.

Furthermore, since the pneumatic unit can be made simple and inexpensive to manufacture compared to a full scale ventilator, the pneumatic unit of the present disclosure, much like conventional air/oxygen mixers of today, can affordably be made available at most intensive care units in hospital environments. Thereby, also more advanced ventilatory treatment can be made readily available at intensive care units without the need for full scale ventilators.

In general, the capability of providing CPAP-based therapy and, additionally, flow oxygen therapy when the pneumatic unit is operated separately in the unpaired state of operation is sufficient in order to provide adequate ventilatory treatment of neonatal patients. This fact, together with the non-complex functionality and the simple and compact design of the pneumatic unit makes it particularly suitable for use in the treatment of neonatal patients, e.g. neonatal patients in neonatal intensive care units (NICU).

Thus, according to the invention there is provided a modular monitoring and ventilation system comprising:
- a standalone monitor unit for monitoring patient-related parameters indicative of the physiological status of a patient, configured to receive at least one signal indicative of such a patient-related parameter from at least one sensor, and to display information related to said signal on a display of the monitor unit, and
- a standalone pneumatic unit for ventilatory treatment of a patient through the supply of breathing gas,
wherein the monitor unit and the pneumatic unit are configured to be put in a paired state in which they are communicatively connected to each other in order to exchange information, and to cooperate to provide ventilatory treatment to the patient by controlling the operation of the pneumatic unit based on said at least one signal received by the monitor unit from the at least one sensor and indicative of said monitored patient-related parameter. According to another aspect of the present disclosure there is provided a standalone monitor unit for monitoring patient-related parameters indicative of the physiological status of a patient, configured to receive at least one signal indicative of such a patient-related parameter from at least one sensor, and to display information related to said signal on a display of the monitor unit, wherein the monitor unit is configured to be communicatively connected to a standalone pneumatic unit for ventilatory treatment of a patient through the supply of breathing gas, thereby putting the monitor unit and said pneumatic unit in a paired state in which they are capable of exchanging information, the monitor unit further being configured to cause control of said pneumatic unit to be based on said at least one signal indicative of said monitored patient-related parameter, received by the monitor unit from said at least one sensor.

The sensor from which the standalone monitor unit receives the at least one signal indicative of the patient-related parameter is hence a sensor which together with the monitor unit constitutes a standalone monitoring system for registering signals indicative of a patient-related parameter and displaying information related to said patient-related parameter to a system operator. To this end, the sensor is configured to pick up signals indicative of the patient-related parameter from the patient and to transmit said signals to the monitor unit, typically but not necessarily via a wired signalling line connecting the sensor to a signal input forming a sensor port of the monitor unit. The sensor detecting the patient-related parameter is hence a sensor of the monitoring system and not a sensor of the pneumatic unit or any other medical device.

According to yet another aspect of the present disclosure there is provided a standalone pneumatic unit for providing ventilatory treatment to a patient through the supply of breathing gas, wherein said pneumatic unit is configured to be communicatively connected to a standalone monitor unit for monitoring patient-related parameters indicative of the physiological status of said patient and configured to receive at least one signal indicative of such a patient-related parameter from at least one sensor, thereby putting the pneumatic unit and said monitor unit in a paired state in which they are capable of exchanging information, the pneumatic unit further being configured to control its operation based on said at least one signal indicative of said monitored patient-related parameter, received by the monitor unit from said at least one sensor.

The standalone monitor unit is an Edi monitoring unit, meaning that it is configured to receive, from a bioelectric sensor connected to a signal input of the monitor unit, an EMG signal or another signal indicative of an Edi signal representative of the patient's efforts to breathe. The Edi monitoring unit may further be configured to calculate an Edi signal from the Edi-related signal received from the bioelectric sensor, and to display a graphical representation of the Edi signal or an Edi-related signal on the display of the monitor unit. The bioelectric sensor may be any type of sensor or sensor arrangement configured to obtain EMG signals or other Edi-related signals from the patient, e.g. a sensor arrangement of an oesophageal catheter inserted into the trachea of the patient, well known in the art of neutrally adjusted ventilatory assist (NAVA) ventilation.

When the Edi monitoring unit is paired with the pneumatic unit, the Edi monitoring unit and the pneumatic unit cooperate to control the operation of the pneumatic unit based on said Edi signal.

Thus, according to the invention, the proposed modular system comprises a standalone Edi monitoring unit for monitoring an Edi signal indicative of the breathing efforts of a patient, and a standalone pneumatic unit as described above, wherein the Edi monitoring unit and the pneumatic unit are configured to be put in a paired state in which they exchange information with each other to provide NAVA ventilation to the patient by controlling the pneumatic unit based on said Edi signal.

The modular system of the present invention hence comprises a standalone monitor unit which when operated separately is capable of monitoring patient-related parameters but incapable of providing any type of ventilatory therapy to the patient, and a standalone pneumatic unit which when operated separately is capable of providing only basic ventilatory treatment to the patient, such as CPAP and high flow oxygen therapy, but incapable of providing more advanced ventilatory treatment to the patient, such as NAVA therapy. When the two standalone units are put in a paired state, however, they cooperate to permit more advanced ventilatory treatment, including NAVA therapy, to be provided to the patient.

According to the invention, the modular system is configured to control the operation of the pneumatic unit based on the Edi signal such that a measured pressure, typically a proximal pressure substantially corresponding to the airway pressure of the patient, is adjusted in dependence of said Edi signal. This enables efficient NAVA treatment to be provided to the patient.

Thus, according to the invention there is provided a modular monitoring and ventilation system comprising:
- a standalone Edi monitoring unit configured to receive a bioelectric signal indicative of an Edi signal of a monitored patient from a bioelectric sensor, and to display a graphical representation of said Edi signal or an Edi-related signal on a display of said monitor unit, and
- a standalone pneumatic unit for ventilatory treatment of a patient through the supply of breathing gas, wherein said pneumatic unit, when operated separately in an unpaired state in which it is not paired with said monitor unit, is capable of providing only basic ventilatory treatment to the patient, not including ventilatory treatment based on Edi signals,
wherein the Edi monitoring unit and the pneumatic unit are configured to be put in a paired state in which they are communicatively connected to each other and configured to cooperate to provide ventilatory treatment to the patient by controlling the operation of the pneumatic unit based on said Edi signal.

The standalone monitor unit is configured to, when paired with the pneumatic unit, transmit an Edi signal derived from said bioelectric signal to the pneumatic unit, whereby the pneumatic unit is configured to receive the Edi signal and use it as a control signal in the control of the operation of the pneumatic unit.

According to another aspect of the present disclosure there is provided method for enabling enhanced functionality of a standalone monitor unit for monitoring patient-related parameters indicative of the physiological status of a patient. The method comprises the steps of:
- receiving, in the monitor unit, at least one signal indicative of such a patient-related parameter from at least one sensor;
- displaying, on a display of said monitor unit, information related to said signal;
- communicatively connecting the monitor unit with a standalone pneumatic unit for providing ventilatory treatment to the patient through the supply of breathing gas, thereby putting the monitor unit and said pneumatic unit in a paired state in which they are capable of exchanging information, and
- causing the operation of said pneumatic unit to be based on said at least one signal indicative of the monitored patient-related parameter, received by the monitor unit from said at least one sensor.

In one embodiment the method further comprises the steps of:
- transmitting said signal received by the monitor unit from the at least one sensor and indicative of the monitored patient-related parameter, or a signal derived therefrom, from the monitor unit to the pneumatic unit, and
- receiving the transmitted signal in the pneumatic unit and using it as a control signal in the control of the operation of the pneumatic unit.

As discussed above, the monitor unit may be an Edi monitor and the at least one signal received by the monitor unit from the at least one sensor may be a bioelectric signal indicative of an Edi signal representative of the patient's efforts to breathe, received from a bioelectric sensor.

In one embodiment, the method comprises the steps of:
- obtaining, in the pneumatic unit, measurements of a pressure related to the airway pressure of the patient, and
- controlling the operation of the pneumatic unit such that said pressure is varied in dependence of said Edi signal.

As discussed above, one problem addressed by the present invention is how to minimize the time to provision of adequate ventilatory treatment of a patient connected to a standalone monitor unit, after said monitor unit has indicated the need for such ventilatory treatment, e.g. through the generation of an alarm. It should thus be appreciated that the step of communicatively connecting the monitor unit to the standalone pneumatic unit in order to provide ventilatory treatment to the patient based on the monitored patient-related parameters according to the principles of the present disclosure may be carried out in response to the generation of an alarm by said monitor unit indicating a deviation of said monitored patient-related parameter from an expected value or range.

According to another aspect of the present disclosure, the modular monitoring and ventilation system is configured to transfer information related to operational parameters of the pneumatic unit from the pneumatic unit to the monitor unit when the two units are operated in said paired state. Such information may include parameters relating to an ongoing ventilatory treatment of the patient provided by the pneumatic unit, setting parameters relating to various settings of the pneumatic unit, and/or status parameters indicative of the current status of the pneumatic unit.

According to the invention, when paired with the pneumatic unit, the standalone monitor unit is configured to:
- receive, from said pneumatic unit, information related to an ongoing ventilatory treatment of the patient provided by the pneumatic unit, and
- associatively display, on the display of the monitor unit, information related to the signal indicative of the patient-related parameter monitored by the monitor unit and the information received from the pneumatic unit and relating to the ongoing ventilatory treatment of the patient.

In this context, associatively displaying information means that the different types of information are displayed simultaneously and in relation to each other such that an operator of the system easily can compare the different types of information. Information related to the signal indicative of the patient-related parameter monitored by the monitor unit should in this context be considered to include at least information related to the signal itself, information related to other signals derived from said signal, and information related to the monitored patient-parameter of which said signal is indicative. The displayed information may comprise any type of graphical representation of such signals or patient-related parameter, such as signal curves, symbols or numerical values.

Thus, according to this aspect of the present invention there is provided a modular monitoring and ventilation system comprising:
- a standalone monitor unit for monitoring patient-related parameters indicative of the physiological status of a patient, configured to receive at least one signal indicative of such a patient-related parameter from at least one sensor, and to display information related to said signal on a display of the monitor unit, and
- a standalone pneumatic unit for ventilatory treatment of a patient through the supply of breathing gas,
wherein the monitor unit and the pneumatic unit are configured to be put in a paired state in which they are communicatively connected to each other and in which the monitor unit is configured to receive, from the pneumatic unit, information related to an ongoing ventilatory treatment of the patient provided by the pneumatic unit, and to associatively display, on said display of the monitor unit, information related to the signal indicative of the patient-related parameter monitored by the monitor unit and the information received from the pneumatic unit and relating to the ongoing ventilatory treatment of the patient.

By associatively displaying patient-related information obtained by the monitor unit itself and information received by the monitor unit from the pneumatic unit, information relating to a monitored patient-related parameter can be displayed in association with e.g. information relating to operational parameters of the pneumatic unit. This may be very useful to a physician in order to assess how the ventilatory treatment currently provided to the patient by the pneumatic unit affects the patient-related parameter monitored by the monitor unit, and so how to best adjust the operation of the pneumatic unit in order to optimize the ventilatory treatment.

The monitor unit is configured to associatively display, in a common frame of reference, a graphical representation of a signal indicative of said monitored patient-related parameter and a graphical representation of a signal received by the monitor unit from the pneumatic unit and indicative of an operational parameter of the pneumatic unit.

Said signal indicative of the monitored patient-related parameter is an Edi signal derived from a bioelectric signal picked up from the patient by means of a bioelectric sensor, said signal received from the pneumatic unit is a pressure signal representing a pressure measured by a pressure sensor of the pneumatic unit, typically a proximal pressure substantially corresponding to the airway pressure of the patient, and said common frame of reference is a timeline displayed in a display window of the display, on which timeline said graphical representations are displayed. This renders possible for an operator of the modular system to see how the pressure delivered to the patient follows the detected Edi signal.

According to another aspect of the present disclosure there is provided a method for enabling enhanced functionality of a standalone monitor unit for monitoring patient-related parameters indicative of the physiological status of a patient. The method comprises the steps of:
- monitoring, by means of said standalone monitor unit, a patient-related parameter indicative of the physiological status of the patient;
- providing a standalone pneumatic unit for ventilatory treatment of a patient through the supply of breathing gas;
- putting the monitor unit and the pneumatic unit in a paired state in which they are communicatively connected to each other in order to exchange information, and
- receiving, in the monitor unit, information related to an ongoing ventilatory treatment of the patient provided by the pneumatic unit, from the pneumatic unit, and
- associatively displaying, on a display of the monitor unit, information related to the patient-related parameter monitored by the monitor unit and the information received from the pneumatic unit and relating to the ongoing ventilatory treatment of the patient.

The connection through which the monitor unit and the pneumatic unit are communicatively connected to each other when put in the paired state may be any type of wired or wireless connection, including but not limited to an R232 serial connection, an Ethernet connection or a Bluetooth connection.

Furthermore, the monitor unit and/or the pneumatic unit are preferably provided with a mechanical connector arrangement for mechanically connecting the monitor unit and the pneumatic unit to each other.

In one embodiment, the mechanical connector arrangement is part of a docking interface of the modular system, which docking interface further comprises a signal connector and/or an electric connector for transmission of signals and/or electric power between the monitor unit and the pneumatic unit when the pneumatic unit is docked to the monitor unit via said docking interface.

In one embodiment, the monitor unit is configured to be powered by means of a connection to an external power source, such as a mains supply, and further configured for electrical connection to the pneumatic unit to supply electrical power to the pneumatic unit, e.g. through an electric cable or the above mentioned electric connector arrangement of a docking interface.

Thus, the pneumatic unit is preferably configured to be connected to and receive electrical power from the monitor unit. However, as a precautionary measure that further improves the flexibility of the pneumatic unit, the pneumatic unit is preferably configured to be capable of strict mechanical operation in the meaning of being able to provide at least basic ventilatory treatment to the patient without the supply of electrical power.

Further advantageous aspects of the invention will be described in the detailed description following hereinafter.

### BRIEF DESCRIPTION OF THE DRAWINGS

The present invention will become more fully understood from the detailed description provided hereinafter and the accompanying drawings which are given by way of illustration only. In the different drawings, same reference numerals correspond to the same element.
Fig. 1A illustrates a standalone monitor unit according to an exemplary embodiment of the present disclosure, connected to a patient for monitoring patient-related parameters indicative of the physiological status of the patient;
Fig. 1B illustrates some components and functional modules of the standalone monitor unit shown in Fig. 1A;
Fig. 2A illustrates a standalone pneumatic unit according to an exemplary embodiment of the present disclosure, connected to a patient for providing ventilatory treatment to the patient through the supply of breathing gas;
Fig. 2B illustrates some components and functional modules of the standalone pneumatic unit shown in Fig. 2A;
Fig. 3A illustrates a modular monitoring and ventilation system according to an exemplary embodiment of the present disclosure, connected to a patient for providing ventilatory treatment to the patient based on monitored patient-related parameters, and
Fig. 3B illustrates a graphical user interface of the monitor unit illustrated in Figs. 1A-1B, when operated in a paired state in which it is communicatively connected to the pneumatic unit illustrated in Figs. 2A-2B to form said modular monitoring and ventilation system.

### DETAILED DESCRIPTION

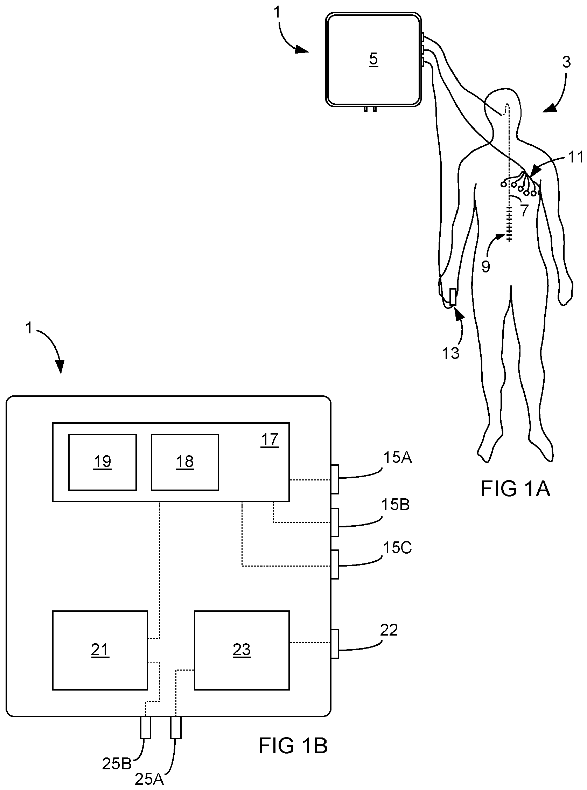

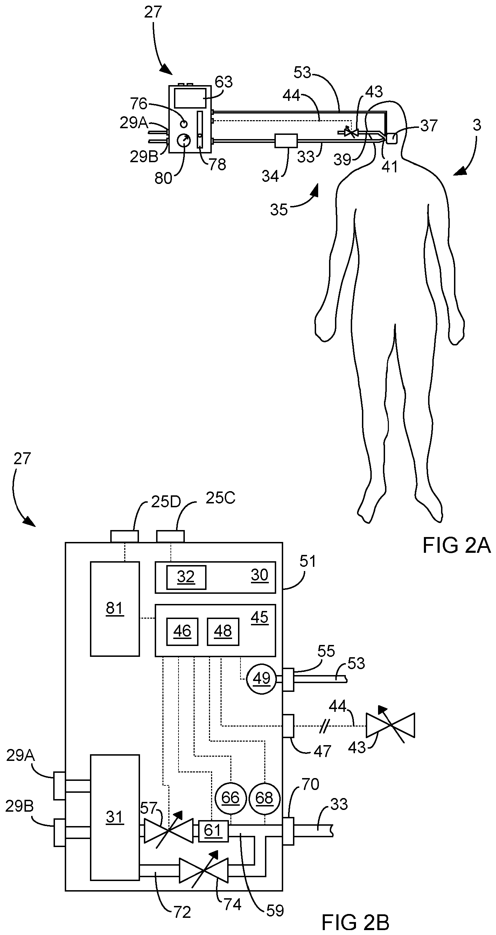

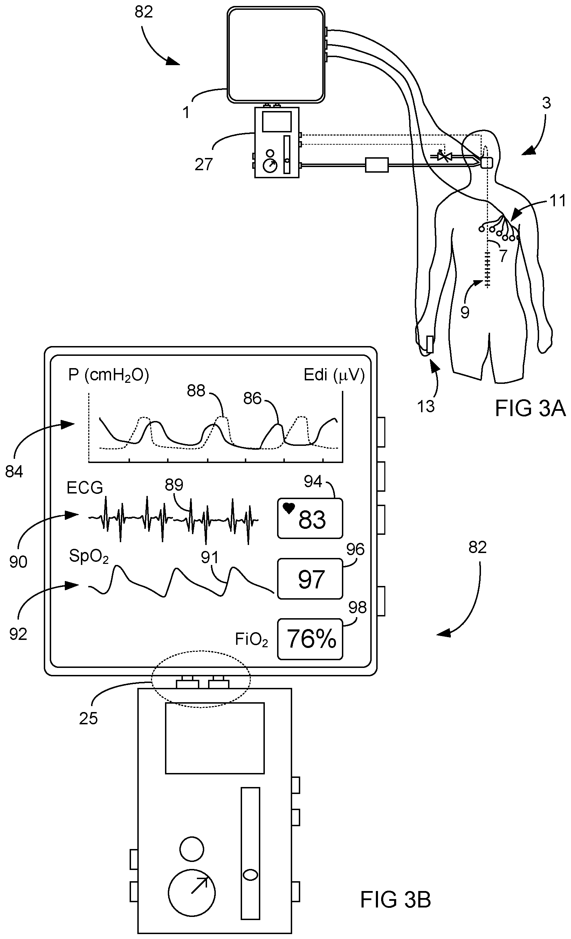

In the following, a modular monitoring and ventilation system of the present disclosure will be described with reference to the accompanying drawings of which Figs. 1A-1B illustrate the standalone monitor unit of the system, Figs. 2A-2B illustrate the standalone pneumatic unit of the system, and Fig. 3A-3B illustrates the modular monitoring and ventilation system wherein said standalone monitor unit and said standalone pneumatic unit are communicatively connected to each other in a paired state in which they cooperate to provide ventilatory treatment to a patient in accordance with the principles of the present invention.

That the monitor unit and the pneumatic unit of the present disclosure are standalone units herein means that they are separate and self-contained units able to function by themselves, without being connected to or integrated in other medical devices.

With reference now made to Fig. 1A, a medical monitor in form of a standalone monitor unit 1 is arranged to monitor various physiological parameters of a patient 3, hereinafter referred to as patient-related parameters. To this end, the monitor unit 1 is configured to receive signals indicative of said patient-related parameters from different sensors to which the monitor unit 1 is connected, and to display information relating to the monitored patient-related parameters on a display 5 of the monitor unit.

The display 5 of the monitor unit 1 may for example be a touch screen forming a graphical user interface (GUI) through which an operator of the monitor unit 1 can change settings of the monitor unit 1, e.g. in order to select the information to be displayed on the display 5, change alarm settings for monitored patient-related parameters, etc.

The monitor unit 1 is a standalone Edi monitoring unit, meaning that it is configured to monitor the Edi of the patient. To this end, the monitor unit 1 is configured to receive bioelectric signals representative of the Edi from a bioelectric sensor 7 arranged to pick up said bioelectric signals from the patient 3, and to display information relating to the Edi signal on said display 5.

The act of taking a breath is controlled by the respiratory centre of the brain, which decides the characteristics of each breath, timing and size. The respiratory centre sends a signal along the phrenic nerve, excites the diaphragm muscle cells, leading to muscle contraction and descent of the diaphragm dome. As a result, the pressure in the airway drops, causing an inflow of air into the lungs. As well known in the art, the Edi signal reflects the electrical activity of the diaphragm and so the patient's effort to breathe in accordance with the signals received from the respiratory centre of the brain.

This fact is used in the field of NAVA ventilation, which is a mode of mechanical ventilation in which the electrical activity of the diaphragm is captured, fed to a NAVA-enabled ventilator and used to assist the patient's breathing in synchrony with and in proportion to the patient's own breathing efforts.

In NAVA, the patient's breathing efforts are typically sensed by measuring the electromyogram (EMG) of the contracting diaphragm, sometimes referred to as diaphragm EMG. The EMG signals are then processed in various ways and a signal representative of the Edi is calculated and used in the control of the NAVA-enable ventilator, typically by controlling the supply of breathing gas to the patient in synchrony and in proportion to the Edi.

In the embodiment illustrated in Fig. 1A, the bioelectric sensor 7 is an oesophageal catheter provided with an array of electrodes 9 for detecting EMG signals representative of the Edi of the patient 3 when the catheter is inserted into the oesophagus of the patient 3. Such catheters are often used to register the EMG signals used to control NAVA-enabled ventilators and are therefore often referred to as NAVA catheters. Besides EMG components, the raw signals picked up by the electrodes 9 of the NAVA catheter typically comprises ECG components which can be extracted from said raw signals in order to display information relating to the ECG of the patient 3 on the display 5. Also the heart rate of the patient 3 may be determined from the signals captured by the NAVA catheter, and displayed on the display 5. Furthermore, the NAVA catheter may be equipped with a temperature sensor in order to sense the temperature of the patient 3 when the NAVA catheter is inserted into the oesophagus of the patient 3, whereby information related to the sensed temperature may be displayed on the display 5 of the monitor unit 1.

In another embodiment (not shown), the bioelectric sensor 7 for picking up signals representative of said Edi signal may be realized in form of a set of chest wall surface electrodes for recording the diaphragm EMG from the surface of the skin of the patient 3. It should thus be appreciated that the bioelectric sensor 7 may be realized in different forms as long as it is capable of detecting a bioelectric signal indicative of the Edi of the patient 3.

The NAVA technology is further described in e.g. WO 1998/48877, WO 1999/62580, WO 2006/131149, and WO 2008/131798.

The monitor unit 1 of the present disclosure is thus configured to receive the signals picked up by the bioelectric sensor 7, determine from said signals information relating to the Edi signal of the patient 3, and display information related to said Edi signal and hence related to the breathing efforts of the patient 3 on the display 5 of the monitor unit 1.

The Edi of the patient is hence one patient-related parameter monitored by the standalone monitor unit 1. Other examples of patient-related parameters which may be monitored by the standalone monitor unit 1 is the electrocardiogram (ECG) and peripheral capillary oxygen saturation (SpO₂) of the patient 3. To this end, the monitor unit 1 may be configured to receive signals indicative of the ECG of the patient 3 from a sensor arrangement 11, e.g. a set of electrodes attached to the surface of the skin of the patient 3, and to receive signals indicative of the SpO₂ of the patient 3 from another sensor arrangement 13, e.g. a pulse oximeter device, and to display information related to the ECG and the level of SpO₂ of the patient 3 on the display 5, e.g. in form of signal curves and/or numeric values.

Besides monitoring various patient-related parameters, the standalone monitor unit 1 may be configured to monitor the position of the NAVA catheter 7 used for picking up the Edi-related EMG signals, and to generate an alarm in case of improper catheter position or in case a monitored patient-related parameter deviates from a desired value or range. The position of the NAVA catheter 7 relative to the centre of the electrically active region, Ear(di), of the diaphragm can be determined by the monitor unit 1 by comparing the amplitudes and/or the polarities of the signals picked up by a plurality of electrode pairs in the array of electrodes 9, as well known in the art of NAVA. Preferably, the displayed information relating to the position of the NAVA catheter comprises a graphical representation of the NAVA catheter, e.g. in form of a vertically elongated object, and its position in relation to the diaphragm of the patient. For example, an object in form of a circle or the like, representing the diaphragm of the patient 3, may be displayed at a position along said vertically elongated object corresponding to the diaphragm's position relative to the NAVA catheter, as determined from the signals picked up by the NAVA catheter.

Fig. 1B illustrates schematically some components and logical modules of the standalone monitor unit 1 and will now be described with simultaneous reference to Fig. 1A.

First, the monitor unit 1 comprises a plurality of signal inputs 15A-15C for receiving the signals indicative of the monitored patient-related parameters and picked up by the various sensors. Here, a first signal input 15A is configured to receive the Edi-related EMG signals registered by the bioelectric sensor 7, a second signal input 15B is configured to receive the ECG signals registered by the ECG sensor arrangement 11, and a third signal input 15C is configured to receive signals indicative of the level of SpO₂ of the patient 3 registered by the SpO₂ sensor arrangement 13.

The signals received via the signal inputs 15A-15C are forwarded to a control unit 17 of the monitor unit 1. The control unit 17 comprises a processing unit 18, e.g. in form of a microprocessor, which is configured to process said signals in various ways and to cause display of information relating to the received signals, other signals derived from said received signals, or information relating to the patient-related parameters of which said received signals are representative, on the display 5 of the monitor unit.

For example, the control unit 17 is configured to receive the EMG signals from the signal input 15A, calculate an Edi signal representative of the breathing efforts of the patient 3 from said EMG signals, and display a signal curve representing the Edi signal on the display 5 of the monitor unit, as will be further described below with reference to Fig. 2C.

The control unit 17 is further seen to comprise a digital storage unit 19, e.g. in form of a non-volatile memory, storing a computer program comprising computer-readable code which when executed by the processing unit 18 of the control unit 17 causes the monitor unit 1 to perform the actions and method steps described herein as being performed in or by the monitor unit 1.

Furthermore, the monitor unit 1 comprises a communication module 21 for communicating with a standalone pneumatic unit when the monitor unit 1 and the pneumatic unit are put in a paired state in which they are communicatively connected to each other to form the modular monitoring and ventilation system of the present disclosure, as will be described in more detail below with reference to Figs. 3A and 3B.

The communication module 21 is configured to transmit information to the pneumatic unit, e.g. information obtained by the monitor unit 1 and relating to monitored patient-related parameters and additionally also operational parameters of the monitor unit 1, such as monitor unit settings. In particular, the communication module 21 is configured to transmit the Edi signal or an Edi-related signal, derived by the control unit 17 from the EMG signals picked up by the bioelectric sensor 7, to the pneumatic unit in order to permit the pneumatic unit to control ventilatory treatment of the patient 3 based on said Edi or Edi-related signal by controlling a supply of breathing gas to the patient 3 in dependence of said Edi or Edi-related signal.

The communication module 21 of the monitor unit 1 is further configured to receive from the pneumatic unit information obtained by the pneumatic unit and relating to an ongoing ventilatory treatment of the patient 3, e.g. pressure and flow measurements obtained by sensors of the pneumatic unit, pneumatic unit settings etc.

The control unit 17 of the monitor unit 1 may be configured to use the information received from and obtained by the pneumatic unit in various ways. In particular, the control unit 17 is configured to cause associated display of information received from the pneumatic unit and related to an ongoing ventilatory treatment of the patient 3 and information related to at least one patient-related parameter monitored by the monitor unit 1, on the display 5 of the monitor unit 1, as will be described in more detail below with reference to Fig. 3B.

Furthermore, the monitor unit 1 is seen to comprise a power input 22 and a power module 23 for receiving electrical power from an external power source (not shown), such as the mains supply of a hospital facility in which the monitor unit is located, and for powering both internal electrical components of the monitor unit 1 and the pneumatic unit when said pneumatic unit is electrically connected to the monitor unit 1 via an electric cable or an electric connector 25A of a docking interface through which the monitor unit 1 and the pneumatic unit can be mechanically and electrically connected to each other.

Besides an electric connector 25A for the supply of electric power to the pneumatic unit, said docking interface of the monitor unit 1 comprises a signal connector 25B for transmission of the above mentioned information relating to monitored patient-related parameters to the pneumatic unit, including at least said Edi or Edi-related signal for use in the control of the pneumatic unit, and additionally also for reception of the above mentioned information relating to an ongoing ventilatory treatment of the patient, transmitted to the monitor unit 1 by the pneumatic unit.

The pneumatic unit will now be described with reference to Figs. 2A-2B.

Fig. 2A illustrates a standalone pneumatic unit 27 for providing ventilatory treatment to the patient 3 through the supply of breathing gas, according to an exemplary embodiment of the present disclosure.

The pneumatic unit 27 is a small-sized, portable, standalone unit which is capable of providing only basic ventilatory treatment of a patient when operated separately in an unpaired state of operation in which it is not communicatively connected to the monitor unit 1 shown in Figs. 1A-1B. The pneumatic unit 27 is hence not a full scale ventilator capable of providing advanced ventilatory treatment to the patient when operated in the unpaired state. In particular, the pneumatic unit 27 is incapable of controlling its operation based on Edi signals or Edi-related signals indicative of the patients breathing efforts when operated in said unpaired state of operation.

According to one embodiment, the pneumatic unit 27 is a standalone CPAP device configured to provide a continuous positive airway pressure to the patient 3, which continuous positive airway pressure may be set by an operator of the pneumatic unit via pressure adjusting means (not shown) of the pneumatic unit, e.g. in form of a touch button of a touch screen, a mechanical rotary knob or the like. The CPAP device may be configured to provide different types of non-invasive positive pressure ventilation (NIPPV) to the patient 3, such as conventional CPAP therapy or bilevel/biphasic positive airway pressure (BiPAP) therapy, the latter being a ventilation mode in which the delivered pressure follows a certain pattern according to which it is altered between two substantially constant pressure levels. Additionally, in another mode of operation, the CPAP device may be configured to provide high flow therapy to the patient 3 by allowing an operator of the pneumatic unit 27 to set a continuous flow of breathing gas to be supplied to the patient 3, e.g. a flow within the range of 2 to 30 I/min.

The configuration and operation of the pneumatic unit 27 will now be described with simultaneous reference to Figs. 2A and 2B.

The pneumatic unit 27 comprises at least two gas inlets, namely a first gas inlet 29A for receiving pressurized oxygen from an external pressurized oxygen source (not shown) and a second gas inlet 29B for receiving pressurized air from an external pressurized air source (not shown). Typically, the pneumatic unit 27 is adapted for connection to wall outlets for pressurized oxygen and air, commonly found in hospital facilities.

The flows of air and oxygen received via said gas inlets 29A, 29B are mixed in a gas mixing unit 31 of the pneumatic unit 27 to form a breathing gas mixture which is supplied to the patient 3 via an inspiratory line 33 of a patient circuit 35 through which the pneumatic unit 27 is connected to the patient 3. A humidifier 34 may be arranged in the inspiratory line 33 to humidify the breathing gas before being supplied to the patient 3. The inspiratory line 33 is connected to the airways of the patient 3 via a patient interface 37, which may comprise a nasal cannula, nasal prongs, a face mask or any other type of patient connector known in the art. The patient interface 37 is further connected to an expiratory line 39 of the patient circuit 35 via a Y-piece 41, which Y-piece connects the inspiratory line 33, the expiratory line 39 and the patient interface 37.

The pneumatic unit 27 further comprises an electrically controlled expiratory valve 43 disposed in said expiratory line 39 of the patient circuit 35, and a valve control line 44 through which control signals for controlling said expiratory valve 43 can be transmitted from a control unit 45 of the pneumatic unit 27 via a valve control signal output 47. Furthermore, the pneumatic unit 27 comprises a pressure sensor 49 for measuring a proximal pressure in the patient circuit 35, i.e. a patient pressure substantially corresponding to the airway pressure of the patient 3, and for communicating said patient pressure to the control unit 27. In the illustrated embodiment, the pressure sensor 49 is disposed inside a housing 51 of the pneumatic unit 27 and brought into gaseous connection with the Y-piece 41 of the patient circuit 35 through a pressure gas line 53 connected between the Y-piece 41 and a patient pressure gas inlet 55 of said housing 51. In other embodiments, the pressure sensor 49 may be disposed in said Y-piece 41 and configured to communicate the measured pressure to the control unit 45 of the pneumatic unit by means of a wired or wireless signal connection.

The control unit 45 of the pneumatic unit 27 comprises a processing unit 46, e.g. in form of a microprocessor, and a digital storage unit 48, e.g. in form of a non-volatile memory, storing a computer program comprising computer-readable code which when executed by the processing unit 46 of the control unit 45 causes the pneumatic unit 27 to perform the actions and method steps described herein as being performed in or by the pneumatic unit 27.

Although the expiratory valve 43 has been described above as electrically controlled, the expiratory valve 43 may also be realized in form of a pneumatic expiratory valve that is pneumatically controlled by means of the control unit 45. In this scenario, the control unit 45 may be configured to regulate the expiratory pressure against which the patient 3 exhales by controlling a pressure applied to a valve member of said pneumatic expiratory valve, as well known in the art.

The pneumatic unit 27 further comprises a power module 30 for energizing the electrical components of the pneumatic unit 27. In the embodiment illustrated in Figs. 2A and 2B, the power module 30 comprises an energy storage means 32 in form of a battery or battery pack, which allows the pneumatic unit 27 to be electrically operated also when not electrically connected to the monitor unit 1 or to any other external power source, such as a mains supply of a hospital facility. As will be discussed below, however, the pneumatic unit is also configured to be operated in a strict mechanical mode of operation should the pneumatic unit run out of said battery.

When the pneumatic unit 27 is electrically operated, the operator thereof may set a desired pressure for CPAP therapy, e.g. through the above mentioned pressure adjusting means of the pneumatic unit 27. The control unit 45 may then, in one embodiment, be configured to regulate both an electrically controlled inspiratory valve 57 disposed in a primary inspiratory limb 59 of the pneumatic unit and the expiratory valve 43 disposed in the expiratory line 39 of the patient circuit 35 in order to keep said patient pressure substantially constant and equal to the desired CPAP pressure set by the operator. Regulating not only the expiratory valve 43 but also the inspiratory valve 57 during CPAP treatment makes it possible to decrease the flow through the inspiratory line during expiration of the patient 3, which may be advantageous and sometimes even required in order to maintain the patient pressure substantially equal to the set CPAP pressure also during expiration of the patient. In another embodiment, the control unit 45 may be configured for more conventional CPAP control by opening the inspiratory valve 57 to provide a constant flow of breathing gas through the inspiratory line 33 during both inspiration and expiration phases while only regulating the expiratory valve 43 to keep the patient pressure substantially constant and equal to the desired CPAP pressure.

Furthermore, the pneumatic unit 27 comprises an oxygen sensor 61 disposed in said primary inspiratory limb 59 and coupled to the control unit 45. The oxygen sensor is configured to measure the oxygen content in the breathing gas mixture delivered by the mixing unit 31, and to communicate said oxygen content to the control unit 45 in order for the control unit 45 to cause display of information relating to said oxygen content on a display 63 of the pneumatic unit, e.g. the fraction of inspired oxygen, FiO₂.

Furthermore, the pneumatic unit 27 comprises a second pressure sensor 66 configured to measure a pressure in said primary inspiratory limb 59, and to communicate said pressure to the control unit 45. During normal operation of the pneumatic unit and under non-faulty conditions, this inspiratory limb pressure should correspond approximately to the patient pressure measured by the first pressure sensor 49. As a safety feature of the pneumatic unit 27, the control unit 45 may be configured to compare the pressure in the primary inspiratory limb 59, measured by said second pressure sensor 66, with the patient pressure measured by the first pressure sensor 49, and to generate an alarm, e.g. in form of a visual alarm displayed on the display 63 and/or an audible alarm output on a loudspeaker (not shown) of the pneumatic unit 27, notifying an operator of the pneumatic unit 27 of a faulty condition should the two pressures deviate too much from each other.

Yet further, the pneumatic unit 27 comprises a flow sensor 68 configured to measure the flow of the breathing gas mixture through the primary inspiratory limb 59, between the mixing unit 31 and a breathing gas outlet 70 of the pneumatic unit 27, and to communicate the measured flow to the control unit 45. The control unit 45 is configured to cause display of information of said measured flow on the display 63 of the pneumatic unit 27 and, optionally, to regulate the inspiratory valve 57 based on said measured flow, at least during high flow therapy operation of the pneumatic unit 27 in order to maintain the inspiratory flow of breathing gas at level substantially corresponding to the high flow therapy flow set by an operator.

Besides information related to the oxygen content measured by the oxygen sensor 61 and the flow measured by the flow sensor 68, the control unit 45 is typically configured to cause display of information related to the patient pressure measured by the pressure sensor 49 on the internal display 63 of the pneumatic unit 27. Yet further, the control unit 45 may be configured to cause display of information, on the display 63 of the pneumatic unit 27, related to pressure and/or flow alarms, which alarms may be set by an operator of the pneumatic unit 27 via said display 63,

From the above it should be appreciated that when not electrically connected to the monitor unit 1 or any other external power source, the internal energy storage means 32 may still offer electric operation of the pneumatic unit 27.

However, as mentioned above, the pneumatic unit 27 may also be operated in a strict mechanical mode of operation. To this end, the pneumatic unit comprises a secondary inspiratory limb 72 for conveying breathing gas between the gas mixing unit 31 and the breathing gas outlet 70 in said mechanical mode of operation. In this way, the supply of breathing gas to the patient 3 is independent on the electrically controlled inspiratory valve 57. Instead, the secondary inspiratory limb 72 comprises a strictly mechanical inspiratory valve 74, the opening degree of which can be adjusted by means of a mechanical flow adjustment device 76 of the pneumatic unit, e.g. in form of a rotary control knob, through which the operator of the pneumatic unit 27 can set a desired flow of breathing gas to be provided to the patient 3. In order to operate in a strict mechanical mode of operation, the mixing unit 31 may be a mechanical gas mixing unit in which the gas inlet valves for controlling the supply of gases to be mixed in the mixing unit 31 are mechanically controlled. However, it should be appreciated that the mixing unit 31 may also be an electrical mixing unit whose operation is electronically controlled.

Also, the pneumatic unit 27 is seen to include a mechanical flowmeter 78 for indicating to an operator the current flow of breathing gas supplied to the patient 3 when the pneumatic unit 27 is operated in the mechanical mode of operation. In this embodiment, the mechanical flowmeter 78 is seen to be a flowmeter of the type having a float ball indicating the current flow of breathing gas through the position of the float ball within a metering tube, as well known in the art of air/oxygen mixer units.

Furthermore, the pneumatic unit 27 comprises mechanical oxygen adjustment means 80 for varying the oxygen concentration in the breathing gas mixture of air and oxygen supplied to the patient 3 in the mechanical mode of operation of the pneumatic unit 27, and thus the fraction of inspired oxygen FiO₂.

When operated in a mechanical mode of operation, the pneumatic unit 27 can be operated to provide high flow therapy to the patient by setting a desired flow of breathing gas to be provided to the patient via said flow adjustment device 76, and a desired level of FiO₂ via said oxygen adjustment means. This mode of operation can also be used for applications in which the pneumatic unit 27 serves as an external gas source for delivery of pressurized breathing gas to other medical devices. Furthermore, the pneumatic unit 27 may be configured to provide CPAP treatment to the patient 3 also when operated in the strict mechanical mode of operation. This may be achieved by connecting a bubble pressure generator to the pneumatic unit 27 to form a so called bubble CPAP device, or by connecting a jet device to the pneumatic unit 27 to form a so called jet CPAP device, both of which are capable of providing CPAP treatment in a strict mechanical mode of operation.

The pneumatic unit 27 further comprises an electric connector 25C and a signal connector 25D configured to be connected to the electrical connector 25A and the signal connector 25B, respectively, of the monitor unit 1 when the pneumatic unit 27 is docked to the monitor unit via the above-mentioned docking interface, as will be further described below with reference to Figs. 3A-3B. Yet further, just like the monitor unit 1, the pneumatic unit 27 comprises a communication module 81 configured for communication with the communication module 21 of the monitor unit 1 for exchange of information relating to the patient-related parameters monitored by the monitor unit 1, the ventilatory treatment provided by the pneumatic unit 27, etc.

Figs. 3A and 3B illustrate a modular monitoring and ventilation system 82 according to an exemplary embodiment of the present disclosure.

Here, the standalone monitor unit 1 described above with reference to Figs. 1A-1B and the standalone pneumatic unit 27 described above with reference to Figs. 2A-2B are put in a paired state in which they are communicatively connected to each other to cooperate in order to provide enhanced ventilatory treatment of the patient 3.

In this exemplary embodiment, the monitor unit 1 and the pneumatic unit 27 are communicatively connected to each other through the signal connectors 25A and 25C (see Figs. 1B and 2B), which are brought into signal connection with each other via the above-mentioned docking interface, denoted by reference numeral 25 in Fig. 3B, by docking the pneumatic unit 27 to the monitor unit 1.

It should, however, be appreciated that the communication connection through which the monitor unit 1 and the pneumatic unit 27 are communicatively connected to each other in the paired state of operation could be any suitable type of wired or wireless connection, e.g. a serial connection, an Ethernet connection, a Bluetooth connection or the like.

Furthermore, in this embodiment, the pneumatic unit 27 and the monitor unit are electrically connected to each other through the electric connectors 25B and 25D (see Figs. 1B and 2B), which are brought into electric connection with each other via the docketing interface 25, when docking the pneumatic unit 27 to the monitor unit 1.

It should, however, be appreciated that the electric connection between the monitor unit 1 and the pneumatic unit 27 could be achieved by means of any suitable type of electric connection, e.g. by means of an electric cable connecting the power module 23 of the monitor unit 1 with the power module 30 of the pneumatic unit 27. When electrically connected to the monitor unit, electric energy is supplied to the power module 30 of the pneumatic unit 27 from the power module 23 of the monitor unit 1 in order to energize the electrical components of the pneumatic unit 27. Preferably, the power module 30 of the pneumatic unit 27 is configured to charge the internal energy storage means 32 of the pneumatic unit 27 when receiving electrical energy from the monitor unit 1, making the monitor unit 1 serve as a battery charger of the pneumatic unit 27 when the monitor unit 1 and the pneumatic unit 27 are electrically connected to each other.

In the illustrated embodiment, the signal connectors 25B, 25D and the electric connectors 25A, 25C of the docking interface 25 serve the additional purpose of acting as mechanical connectors mechanically connecting the pneumatic unit 27 with the monitor unit. This is achieved by forming the signal and electric connectors 25A, 25B of the monitoring unit 1 as male connectors, forming the signal and electric connectors 25C, 25D of the pneumatic unit 27 as female connectors, and adapting the sizes of said male and female connectors such that the pneumatic unit 25 is fixed in relation to the monitor unit 1 through frictional locking of said male-type of connectors 25A, 25B of the monitor unit 1 by said female-type of connectors 25C, 25D of the pneumatic unit 27, when brought into sliding engagement with each other by docking the pneumatic unit 27 to the monitor unit 1.

It should be appreciated that mechanical connection between the monitor unit 1 and the pneumatic unit 27 may be achieved also in other ways. For example, the monitor unit 1 and the pneumatic unit 27 may comprise mechanical connectors configured to fixedly connect the two units to each other in a side-by-side manner instead of the vertically stacked manner illustrated in Figs. 3A and 3B.

The monitor unit 1 and the pneumatic unit 27 are particularly intended to be put in said paired state when the need arises for ventilatory treatment of a patient whose medical status is monitored by means of the standalone monitor unit 1. For example, when a patient-related parameter monitored by the standalone monitor unit 1 indicates that the patient 3 is in need of supported ventilation, the portable pneumatic unit 27 can be fetched by medical personnel and paired with the monitor unit 1 e.g. by docking the pneumatic unit 27 to the monitor unit 1 via the docking interface 25. Thereby, advanced ventilatory treatment can be provided to the patient 3 on the monitoring site, i.e. without moving the patient 3 or any bulky ventilator.

When operated in said paired state, the operation of the pneumatic unit 1 is controlled based on a patient-related parameter monitored by the monitor unit 1, and transmitted from the monitor unit 1 to the pneumatic unit 27 via the communication connection established therebetween in said paired state. Controlling the operation of the pneumatic unit 1 typically involves control of the inspiratory 57 and/or expiratory 43 valves of the pneumatic unit 27 in order to control at least the supply of breathing gas to the patient.

In a preferred embodiment, said monitored patient-related parameter on which the operation of the pneumatic unit is based is the Edi signal or an Edi-related signal derived by the control unit 17 of the monitor unit 1 based on the bioelectric signals, e.g. in form of diaphragm EMG signals, picked up by the bioelectric sensor 7 from the patient 3.

When the operation of the pneumatic unit 27 is based on said Edi or Edi-related signal, the system 82 constitutes a NAVA-enabled ventilator system providing ventilatory treatment to the patient 3 based on the patient's effort to breathe, as reflected by the Edi signal. How to best control the supply of breathing gas to a patient based on a monitored Edi signal is well known in the art of NAVA ventilation and needs not to be explained in detail herein. The type of NAVA ventilation provided by the pneumatic unit 27 may be any known type of NAVA ventilation but is preferably what is sometimes referred to as cNAVA (continuous NAVA) in the context of NAVA ventilators, meaning that the pneumatic unit 27 is operated such that the patient pressure, e.g. the pressure measured by pressure sensor 49, follows the Edi signal nearly continuously.

Preferably, both the inspiratory valve 57 and the expiratory valve 43 (see Fig. 2B) of the pneumatic unit 27 are controlled based on the Edi or Edi-related signal. In some embodiments, however, only the inspiratory valve 57 is controlled based on the Edi or Edi-related signal in order to adapt the patient pressure to said signal. In yet other embodiment, the inspiratory valve 57 is maintained at a certain degree of opening to make the inspiratory flow of breathing gas substantially constant, whilst only the expiratory valve 43 is controlled based on the Edi or Edi-related signal to adapt the patient pressure to the Edi or Edi-related signal.

From the above it should be understood that, in the paired state of operation, the monitor unit 1 is preferably configured to power the pneumatic unit 27, and to transmit information related to monitored patient-related parameters to the pneumatic unit 27 for the purpose of adapting the operation of the pneumatic unit 27 to the needs of the patient as reflected by said monitored patient-related parameters.

Furthermore, the monitor unit 1 may be configured to transmit setting parameters to the pneumatic unit, allowing an operator of the modular monitoring and ventilation system 82 to adjust settings of the pneumatic unit 27, e.g. operational settings relating to a ventilatory treatment to be provided to the patient 3, via the monitor unit 1. For example, the monitor unit 1 may be configured to allow an operator to set an upper pressure limit and/or a lower pressure limit for the pressure delivered to the patient during the above-mentioned cNAVA ventilation, via a touch screen of the monitor unit 1, whereby the monitor unit 1 may transmit these pressure settings to the pneumatic unit in order for the control unit 45 of the pneumatic unit 27 to adapt the cNAVA ventilation provided by the pneumatic unit 27 to the patient 3 to said pressure limits.

When it comes to transmission of information in the opposite direction, i.e. transmission of information from the pneumatic unit 27 to the monitor unit 1, such information typically includes operational parameters of the pneumatic unit, including but not limited to parameters relating to an ongoing ventilatory treatment of the patient 3 provided by the pneumatic unit 27, setting parameters relating to various settings of the pneumatic unit 27, and status parameters indicative of the current status of the pneumatic unit 27.

Examples of operational parameters transmitted to the monitor unit 1 and relating to an ongoing ventilatory treatment of the patient 3 are the fraction of inspired oxygen, FiO₂, measured by the oxygen sensor 61 (see Fig. 2B) of the pneumatic unit 27, the flow of breathing gas delivered to the patient, measured by the flow sensor 68, the inspiratory pressure measured by the pressure sensor 66, and the patient pressure measured by the pressure sensor 49. Examples of setting parameters that may be transmitted to the monitor unit 1 from the pneumatic unit 27 are alarm settings defining acceptance windows for said operational parameters. Examples of status parameters that may be transmitted to the monitor unit 1 from the pneumatic unit 27 are battery indicator parameters indicating the battery level and/or charging state of the energy storage means 32 of the pneumatic unit 27.

In particular, the pneumatic unit 27 is configured to transmit signals indicative of the patient pressure measured by the pressure sensor 49 to the monitor unit 1, whereby the monitor unit 1 is configured to associatively display information relating to the Edi signal and the patient pressure on the display 5 of the monitor unit.

This is shown in Fig. 3B, illustrating an exemplary embodiment of a GUI of the monitor unit 1. The control unit 17 (see Fig. 1B) of the monitor unit 1 is configured to cause display of a signal curve 86 representing the Edi signal of the patient 3 and a pressure curve 88 representing said patient pressure on a common time axis of a diagram constituting a common frame of reference for the Edi signal and the patient pressure, which common frame of reference allows an operator of the modular monitoring and ventilation system 82 to easily compare the Edi signal with the patient pressure. Said diagram is displayed in a display window 84 of the display 5.

Furthermore, in the exemplary embodiment illustrated in Fig. 3B, the control unit 17 of the monitor unit 1 is further configured to cause display of:
- an ECG signal curve 89, representing the ECG signal obtained by means of the ECG sensor arrangement 11, in a second display window 90 of the display 5,
- a SpO₂ signal curve 91 derived from the SpO₂ measurements obtained by means of the SpO₂ sensor arrangement 13, in a third display window 92 of the display 5,
- the heart rate of the patient 3, obtained by means of the ECG sensor arrangement 11, in a fourth display window 94 of the display 5,
- a value of SpO₂ derived from the SpO₂ measurements obtained by means of the SpO₂ sensor arrangement 13, in a fifth display window 96 of the display 5, and
- a value of FiO₂, measured by the oxygen sensor 61 (see Fig. 2B) of the pneumatic unit 27 and received therefrom via the communication connection established between the pneumatic unit 27 and the monitor unit 1 when operated in the paired state of operation.

It should be noted that the view that is displayed on the display 5 in Fig. 3B is merely an example and that any parameters of interest relating to the patient 3, or the operation, status or settings of any or both of the monitor unit 1 and the pneumatic unit 27 may be displayed on the monitor display 5.

Preferably, the control unit 17 of the monitor unit 1 is configured to select what information to put on display on the display 5 in dependence of whether the monitor unit 1 is operated separately in the unpaired state of operation or in the paired state of operation in which it cooperates with the pneumatic unit 27 to provide ventilatory treatment to the patient 3. Typically, there is a first display view, hereinafter referred to as the monitoring view, which is displayed on the display 5 when the monitor unit 1 is not paired with the pneumatic unit 27, and a second display view, hereinafter referred to as the ventilation view, which is displayed on the display 5 when the monitor unit 1 is paired with the pneumatic unit 27 to provide ventilatory treatment to the patient 3. Of course an operator of the system may be given the opportunity to change display settings in order to adapt any or both of said monitoring view and ventilation view based on personal preferences.

For example, said monitoring view that is displayed on the display 5 in the unpaired state of operation of the monitor unit 1 may correspond to the display view shown in Fig. 3B, with the exception that said monitoring view does not comprise the pressure curve 88 representing the patient pressure measured by the pressure sensor 49 of the pneumatic unit, since this pressure is not communicated to the monitor unit 1 when not paired with the pneumatic unit 27. Said ventilation view that is displayed on the display 5 when the monitor unit 1 is paired with the pneumatic unit 27 may correspond to the display view shown in Fig. 3B, with the exception that said ventilation view further comprises information relating to an ongoing ventilatory treatment of the patient 3 provided by the pneumatic unit 27, e.g. measured pressure and flow values, settings of the pneumatic unit, etc. For example, said ventilation view may comprise information relating to upper and/or lower pressure limits of the patient pressure, the gain of the Edi signal used as control signal for the ongoing ventilatory treatment, etc.

Furthermore, although not illustrated in Fig. 3B, at least said ventilation view and preferably also said monitoring view comprises information related to the position of the NAVA catheter for picking up the Edi-related EMG signals from the patient 3, as discussed above with reference to Figs. 1A-1B.

Although the standalone monitor unit 1, the standalone pneumatic unit 27 and the modular monitoring and ventilation system 82 of the present disclosure have been described above in connection with specific embodiments, they should not be construed as being in any way limited to the presented examples. Instead, it is intended that the scope of the present invention is defined only by the claims following hereinafter.

## Claims

1. A modular monitoring and ventilation system (82) comprising:
- a standalone Edi monitor (1) for monitoring a signal reflecting an electrical activity of a diaphragm of a patient (3), configured to receive from at least one bioelectric sensor (7) at least one bioelectric signal indicative of an Edi signal representative of the patient's efforts to breathe, and to display information related to said signal on a display (5) of the Edi monitor (1), and
- a portable standalone pneumatic unit (27) for ventilatory treatment of a patient (3) through the supply of breathing gas, which standalone pneumatic unit (27), when operated separately, is incapable of providing neurally adjusted ventilatory assist (NAVA) treatment to the patient;
wherein the Edi monitor (1) and the pneumatic unit (27) are configured to be put in a paired state in which they are communicatively connected to each other in order to exchange information and to cooperate to provide NAVA treatment to the patient (3) by controlling the operation of the pneumatic unit (27) based on said at least one bioelectric signal indicative of the Edi signal, received by the Edi monitor (1) from said at least one bioelectric sensor (7), such that a proximal pressure substantially corresponding to an airway pressure of the patient is adjusted in dependence of the Edi signal,
wherein the Edi monitor (1), when paired with the pneumatic unit (27), is configured to receive, from said pneumatic unit (27), information related to an ongoing ventilatory treatment of the patient (3) provided by the pneumatic unit, and associatively display, on the display (5) of the Edi monitor (1), information related to the bioelectric signal indicative of the Edi signal and said information received from the pneumatic unit (27) and relating to the ongoing treatment of the patient (3),
wherein the information received from the pneumatic unit (27) comprises a pressure signal representing said proximal pressure, the Edi monitor (1) being configured to associatively display, in a common frame of reference, a graphical representation of an Edi signal derived from the bioelectric signal and a graphical representation of the pressure signal representing said proximal pressure.

2. The modular monitoring and ventilation system (82) according to claim 1, wherein the pneumatic unit (27) when operated separately in an unpaired state in which it is not paired with the Edi monitor (1) is a portable CPAP device.

## Patentansprüche

1. Modulares Überwachungs- und Belüftungssystem (82) umfassend:
- einen eigenständigen Edi-Monitor (1) zum Überwachen eines Signals, das eine elektrische Aktivität eines Diaphragmas eines Patienten (3) widerspiegelt, ausgelegt zum Empfangen von mindestens einem bioelektrischen Sensor (7) mindestens eines bioelektrischen Signals, das ein Edi-Signal angibt, das die Bemühung des Patienten zu atmen darstellt, und zum Anzeigen von Information, die mit dem Signal auf einer Anzeige (5) des Edi-Monitors (1) verbunden ist, und
- eine tragbare eigenständige pneumatische Einheit (27) zur Beatmungsbehandlung eines Patienten (3) durch die Zufuhr von Atemgas, welche eigenständige pneumatische Einheit (27), wenn sie getrennt betrieben wird, keine neural angepasste Atemhilfe (NAVA)-Behandlung für den Patienten bereitstellen kann;
wobei der Edi-Monitor (1) und die pneumatische Einheit (27) dafür ausgelegt sind, gepaart zu werden, in welchem Zustand sie miteinander kommunikativ verbunden sind, um Information auszutauschen und zusammenzuarbeiten,
um eine NAVA-Behandlung für den Patienten (3) durch Steuerung des Betriebs der pneumatischen Einheit (27) bereitzustellen basierend auf dem mindestens einen bioelektrischen Signal, das das Edi-Signal angibt, empfangen von dem Edi-Monitor (1) vom mindestens einen bioelektrischen Sensor (7), so dass ein proximaler Druck im Wesentlichen entsprechend einem Atemwegdruck des Patienten abhängig von dem Edi-Signal eingestellt wird,
wobei der Edi-Monitor (1), wenn er mit der pneumatischen Einheit (27) gepaart ist, dafür ausgelegt ist von der pneumatischen Einheit (27) Information über eine laufende Beatmungsbehandlung des Patienten (3) zu empfangen, die durch die pneumatische Einheit bereitgestellt wird, und auf der Anzeige (5) des Edi-Monitors (1) Information über das bioelektrische Signal assoziativ anzuzeigen, das das Edi-Signal und die Information, die von der pneumatischen Einheit (27) empfangen wird und die laufende Behandlung des Patienten (3) angeht, angibt,
wobei die Information, die von der pneumatischen Einheit (27) empfangen wird, ein Drucksignal umfasst, das den proximalen Druck darstellt, indem der Edi-Monitor (1) dafür ausgelegt ist, in einem gemeinsamen Bezugsrahmen, eine graphische Darstellung eines Edi-Signals, das aus dem bioelektrischen abgeleitet ist, und eine graphische Darstellung des Drucksignals, das den proximalen Druck darstellt, assoziativ anzuzeigen.

2. Modulares Überwachungs- und Belüftungssystem (82) nach Anspruch 1, wobei die pneumatische Einheit (27), wenn sie in einem ungepaarten Zustand, worin sie mit dem Edi-Monitor (1) nicht gepaart ist, getrennt betrieben wird, eine tragbare CPAP-Vorrichtung ist.

## Revendications

1. Système modulaire de surveillance et de ventilation (82), comprenant :
- un moniteur Edi autonome (1) pour surveiller un signal reflétant une activité électrique d'un diaphragme d'un patient (3), configuré pour recevoir à partir d'au moins un capteur bioélectrique (7) au moins un signal bioélectrique indicatif d'un signal Edi représentatif des efforts du patient pour respirer, et pour afficher des informations relatives audit signal sur un dispositif d'affichage (5) du moniteur Edi (1), et
- une unité pneumatique autonome portable (27) pour le traitement ventilatoire d'un patient (3) par l'alimentation en gaz respiratoire, laquelle unité pneumatique autonome (27), lorsqu'elle est actionnée séparément, est incapable de fournir un traitement d'assistance ventilatoire ajusté de manière neuronale (NAVA) au patient ;
dans lequel le moniteur Edi (1) et l'unité pneumatique (27) sont configurés pour être mis dans un état apparié dans lequel ils sont connectés en communication l'un à l'autre afin d'échanger des informations et pour coopérer pour fournir un traitement NAVA au patient (3) en commandant le fonctionnement de l'unité pneumatique (27) sur la base dudit au moins un signal bioélectrique indicatif du signal Edi, reçu par le moniteur Edi (1) à partir dudit au moins un capteur bioélectrique (7), si bien qu'une pression proximale correspondant essentiellement à une pression des voies respiratoires du patient est ajustée en fonction du signal Edi,
dans lequel le moniteur Edi (1), lorsqu'il est apparié avec l'unité pneumatique (27), est configuré pour recevoir, en provenance de ladite unité pneumatique (27), des informations relatives à un traitement ventilatoire en cours du patient (3) fourni par l'unité pneumatique, et afficher de manière associative, sur le dispositif d'affichage (5) du moniteur Edi (1), des informations relatives au signal bioélectrique indiquant le signal Edi et lesdites informations reçues en provenance de l'unité pneumatique (27) et se rapportant au traitement en cours du patient (3),
dans lequel les informations reçues de l'unité pneumatique (27) comprennent un signal de pression représentant ladite pression proximale, le moniteur Edi (1) étant configuré pour afficher de manière associative, dans une trame de référence commune, une représentation graphique d'un signal Edi dérivé du signal bioélectrique et une représentation graphique du signal de pression représentant ladite pression proximale.

2. Système modulaire de surveillance et de ventilation (82) selon la revendication 1, dans lequel l'unité pneumatique (27) lorsqu'elle est actionnée séparément dans un état non apparié dans lequel elle n'est pas appariée avec le moniteur Edi (1), est un dispositif CPAP portable.
